# EUROPEAN PATENT SPECIFICATION

(11) **EP 3 738 520 B1**
(45) Date of publication and mention of the grant of the patent: **24.01.2024**
(21) Application number: 20173959.6
(22) Date of filing: 11.05.2020
(51) Int. Cl.: A61B 17/04

(54) **TISSUE REPAIR ASSEMBLY**
GEWEBE-REPARATUR-MONTAGE
ENSEMBLE DE RÉPARATION DE TISSU

(30) Priority: 16.05.2019 US 201962848753 P; 26.09.2019 US 201962906301 P
(43) Date of publication of application: 18.11.2020
(73) Proprietor: Smith & Nephew, Inc., Memphis, TN 38116 (US); Smith & Nephew Orthopaedics AG, 6300 Zug (CH); Smith & Nephew Asia Pacific Pte Limited, Singapore 138565 (SG)
(72) Inventor: Cunningham, Matthew Dennis, Mansfield, MA 02048 (US); Fu, Rick, Mansfield, MA 02048 (US); Stauffer, Allison Marie, Mansfield, MA 02048 (US)
(74) Representative: Appleyard Lees IP LLP

(56) References cited:
- WO-A1-2012/096706
- WO-A1-2013/134277
- WO-A1-2018/085663
- US-A1- 2004 122 456
- US-A1- 2016 007 989
- US-A1- 2018 271 523
- US-B2- 9 259 218

## Description

### CROSS-REFERENCE TO RELATED APPLICATIONS

This application claims priority to and benefit of co-pending U.S. Provisional Application No. 62/848,753, filed May 16, 2019, and U.S. Provisional Application No. 62/906,301, filed September 26, 2019, both entitled TISSUE REPAIR ASSEMBLY AND METHODS OF USE THEREOF.

### FIELD

The present disclosure relates generally to tissue repair assemblies for repairing soft tissue and, more particularly, to tissue repair assemblies for closing a wound or tear in fibrous tissue.

### BACKGROUND

Areas in the body where tissue can be surgically reattached to bone or repaired when a tear forms in the tissue include, but are not limited to, the biceps tendon, the lateral collateral ligament in the knee, the medial collateral ligament in the knee, the meniscus in the knee, and the popliteal ligament in the leg. Fibrous tissue wounds, such as muscle, ligament, and meniscal tears, can be repaired arthroscopically using sutures. Traditionally, to close a fibrous tissue wound, a surgeon would insert two needles into the tissue loaded with sutures attached to an anchor, thread the sutures across the wound, and then tie knots to fix the free ends of the sutures within the tissue.

To simplify wound closure and to improve fixation, various types of anchors, and devices for delivering the anchors, have been developed. Some types of devices use two separate actuation members that deploy the anchors in a sequential manner, or a single actuation member that deploys the first anchor then retracts to deploy the second anchor. However, the overall strength of the suture/anchor construct is limited by the strength of the anchors, which are typically made from a polymer such as polyetheretherketone (PEEK).

Soft textile anchors are known in the art, which may consist of a woven braid body and a suture slidably woven through the body. When the suture is tensioned, the suture compresses the anchor body in one direction, causing it to expand in one or more other directions, thus increasing the contact area against the tissue. Since soft anchors are typically made entirely of a textile, they may have a strength advantage over typical PEEK anchors. For example, some soft anchors have a tensile strength of 600N/mm², which is six times the safety factor of typical PEEK anchors. WO2013134277A1 relates to an apparatus including a flexible member and two fixation members that are slidably received on a loop formed by the flexible member; US2018271523A1 relates to anchoring devices that are constructed as preformed knot configurations; US2016007989A1 relates to an anchor assembly including a pair of anchor members and a connector member that attaches the pair of anchor members together.

### SUMMARY

Described herein is a tissue repair assembly for repairing soft tissue consisting of two soft anchors connected by a suture. The invention is defined by claims 1 and 4. Each soft anchor is a hollow, cylindrical woven braid with a suture stitched through opposite sides of the anchor. The suture includes a pre-tied sliding knot located between the anchors. When the anchors are deployed in the body and the knot is reduced toward the tissue, the suture slides through the anchors, causing them to expand radially or fold to form a ball shape or cluster, thus increasing the contact area against the tissue and improving the overall construct strength.

According to the claimed invention, the tissue repair assembly of this disclosure includes a needle and a suture/ anchor construct disposed within a bore of the needle. The suture/anchor construct includes first and second flexible anchors coupled to a suture. The suture includes a first section slidably woven through a first anchor, a second section slidably woven at least partially through the first anchor and at least partially through the second anchor, a third section slidably woven through the second anchor, and at least one sliding knot formed between the first section and the third section. The first and second anchors are deployable from a first configuration to a second configuration within a surgical site when tension is applied to the first knot.

The first section of the suture passes around a first end of the first anchor. The second section of the suture passes around a first end of the second anchor. The second section of the suture passes around a second end of the first anchor and around a first end of the second anchor. In examples, the third section of the suture passes around a second end of the second anchor. In examples, the assembly further includes an additional knot formed in the suture for maintaining at least one of the first and second flexible anchors in the second configuration. In examples, in the first configuration, the first and second anchors are elongate and, in the second configuration, the first and second anchors are compressed axially and extended radially.

According to the claimed invention, a suture/anchor construct of this disclosure includes first and second flexible anchors coupled to a first suture. The first suture includes a first section slidably woven through the first anchor, a second section slidably woven at least partially through the first anchor and at least partially through the second anchor, and a third section slidably woven through the second anchor. The construct also includes a second suture slidably woven through the first anchor, and a third suture slidably woven through the second anchor. The first and second anchors are deployable from a first configuration to a second configuration within a surgical site when tension is applied to the second and third sutures, respectively.

In further examples, the construct includes a first knot formed between the first section and the third section of the first suture. In examples, the second suture passes around a first end of the first anchor. In examples, the second suture includes a second knot for maintaining the first anchor in the second configuration. In examples, the third suture passes around a first end of the second anchor. In examples, the third suture includes a third knot for maintaining the second anchor in the second configuration. In examples, in the first configuration, the first and second anchors are elongate and, in the second configuration, the first and second anchors are compressed axially and extended radially.

Examples of a method of closing a wound in soft tissue of this disclosure include deploying a first flexible anchor of an anchor/suture construct on a first side of a wound in tissue and deploying a second flexible anchor of the anchor/suture construct on a second side of the wound. The second anchor is coupled to the first anchor by a suture. The suture includes a first section slidably woven through the first anchor, a second section slidably woven at least partially through the first anchor and at least partially through the second anchor, a third section slidably woven through the second anchor, and a sliding knot formed between the first section and the third section. The method further includes reducing the sliding knot toward the tissue such that the first flexible anchor and the second flexible anchor are brought closer in proximity to close the wound. The first and second anchors are deployable from a first configuration to a second configuration when tension is applied to the sliding knot.

In further examples, the first section of the suture passes around a first end of the first anchor. In examples, the second section of the suture passes around a first end of the second anchor. In examples, the second section of the suture passes around a second end of the first anchor and around a first end of the second anchor. In examples, the third section of the suture passes around a second end of the second anchor. In examples, the deploying the first and second anchors of the anchor/suture construct comprises deploying the first and second anchors from a bore of a needle of a delivery device.

These and other features and advantages will be apparent from a reading of the following detailed description and a review of the associated drawings. It is to be understood that both the foregoing general description and the following detailed description are explanatory only and are not restrictive of aspects as claimed.

### BRIEF DESCRIPTION OF THE DRAWINGS

The disclosure will be more fully understood by reference to the detailed description, in conjunction with the following figures, wherein:
FIGS. 1A-D illustrate a prior art tissue repair assembly and method of use thereof;
FIGS. 2A and 2B show an example of a soft anchor for use in the tissue repair assembly of this disclosure;
FIG. 3 is an example of a suture/anchor construct of the claimed invention using the soft anchor of FIGS. 2A and 2B; and
FIG. 4 is an alternative example of a suture/anchor construct of this disclosure;
FIG. 5 is an alternative example of a soft anchor of this disclosure;
FIGS. 6A-G illustrate various examples of a suture woven through a textile body in the soft anchor of the present disclosure;
FIGS. 7A and 7B illustrate examples of forming a suture/anchor construct according to the claimed invention using a separate connecting suture and tensioning suture(s);
FIGS. 8A-E illustrate additional examples of a suture woven through a textile body in the soft anchor of the present disclosure; and
FIGS. 9A-D illustrate yet further examples of a suture woven through a textile body in the soft anchor of the present disclosure.

### DETAILED DESCRIPTION

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

As used in the specification and claims, for the purposes of describing and defining the invention, the terms "about" and "substantially" are used to represent the inherent degree of uncertainty that may be attributed to any quantitative comparison, value, measurement, or other representation. The terms "about" and "substantially" are also used herein to represent the degree by which a quantitative representation may vary from a stated reference without resulting in a change in the basic function of the subject matter at issue. "Comprise," "include," and/or plural forms of each are open ended and include the listed parts and can include additional parts that are not listed. "And/or" is open-ended and includes one or more of the listed parts and combinations of the listed parts. Use of the terms "up," "down," and the like is intended only to help in the clear description of the present disclosure and are not intended to limit the structure, positioning and/or operation of the disclosure in any manner.

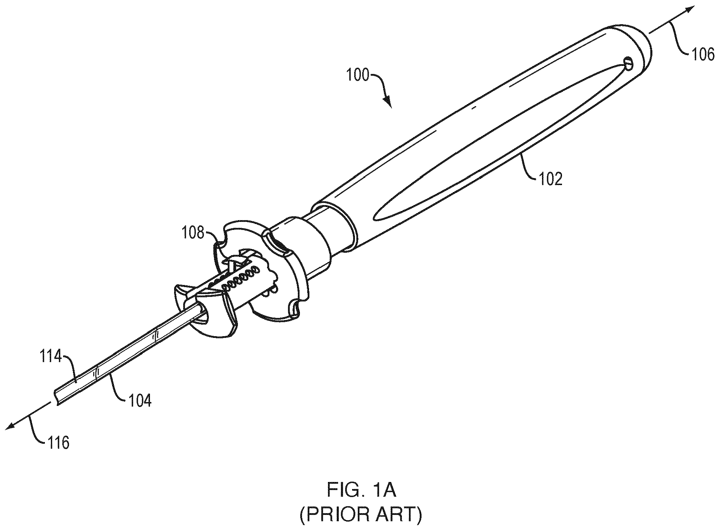

For a better understanding of the present disclosure, FIG. 1A shows an example of a tissue repair assembly 100 for use in, for example, meniscal repair procedures. The assembly 100 generally includes a handle 102 and a needle 104 coupled to the handle 102 and extending along a longitudinal axis 106. In examples, the handle 102 is comprised of plastic and needle 104 is comprised of a biocompatible metal, such as stainless steel or titanium. The handle 102 may include an adjustable depth stop 108 for limiting the depth that the needle 104 may be inserted into a tissue site. The needle 104 may extend from the handle 102 and have an inner surface 114 defining an axial bore 116 extending the length of the needle 104.

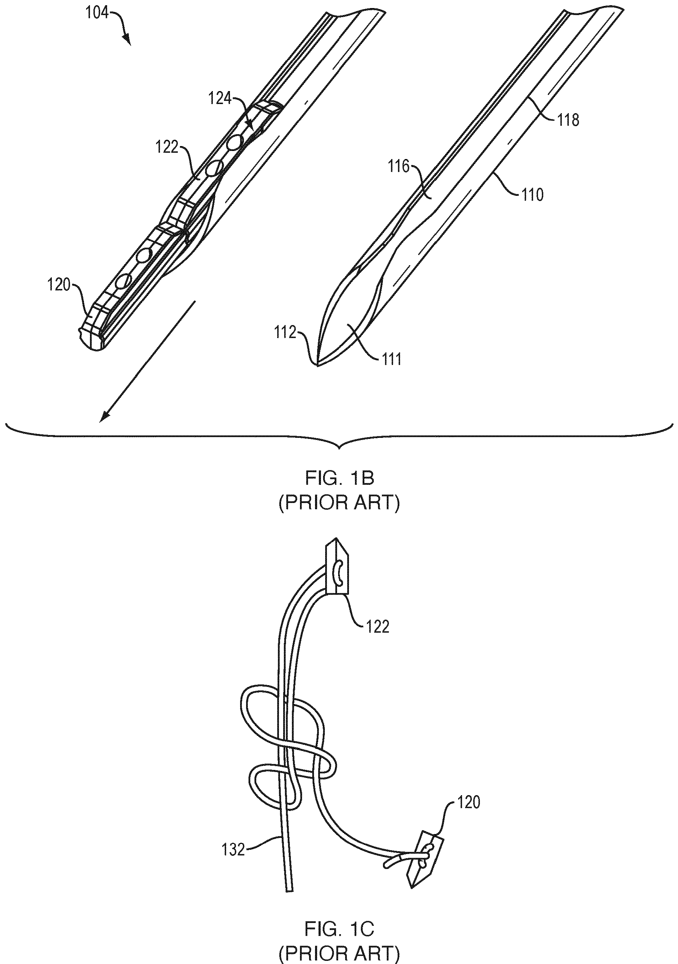

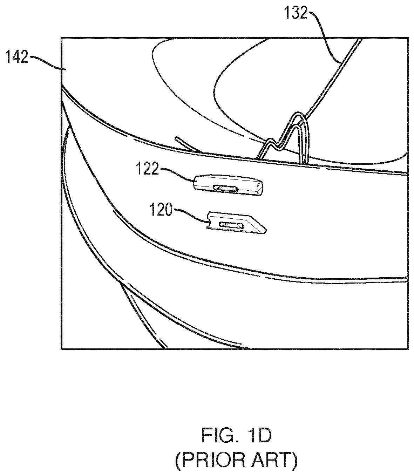

As shown in FIG. 1B, the needle 104 may be configured with a slot 118 extending from an outer surface 110 to the axial bore 116 and to an open distal end 111, which may be beveled to form a pointed, tissue piercing tip 112. The distal end 111 of the needle 104 is configured for at least partially housing a distal anchor 120 and a proximal anchor 122 (both typically formed of PEEK) within the axial bore 116. The proximal anchor 122 and the distal anchor 120 are individually and sequentially deployable from distal end 111 of the needle 104 (for example, with an internal pusher member), and are connected by a length of knotted suture 132, as shown in FIG. 1C. By deploying the proximal anchor 122 and distal anchor 120 on either side of a tear in tissue 142 (FIG. 1D) and tensioning the suture 132, the anchors 120, 122 are brought in closer proximity to each other to close the tear in the tissue 142. Further non-limiting examples of tissue repair assemblies 100 and their methods of use are described in U.S. Publication No. 2018/0116654 to Smith & Nephew, Inc. (Memphis, TN).

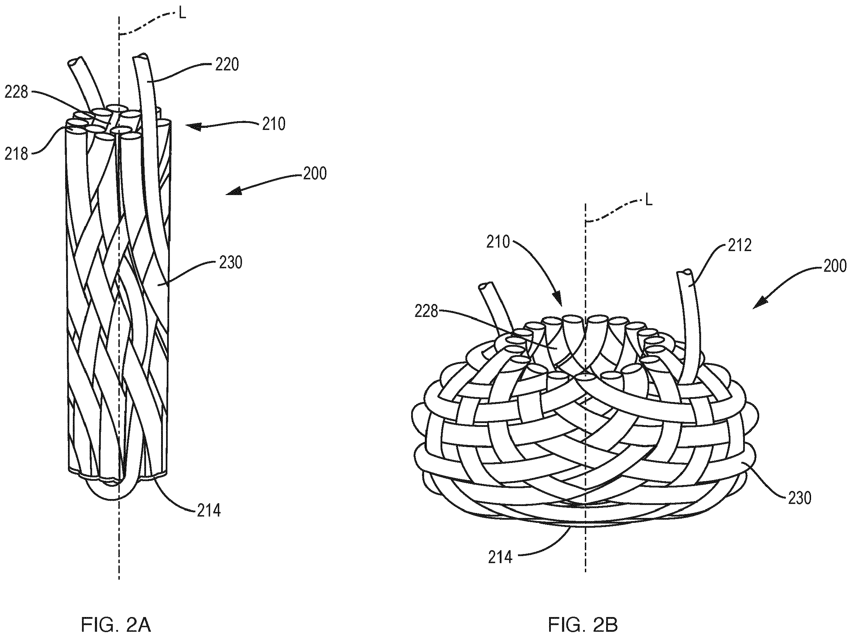

Turning now to FIG. 2A, an example of a soft anchor 200 for use in a tissue repair assembly, such as the tissue repair assembly 100 of FIG. 1, is shown. The soft anchor 200 generally includes a textile body 210 with a suture 212 slidably woven through the sides of the body 210. Examples of the body 210 may be formed from suture, tape, braid or mesh, which may be formed from a bioreabsorbable material. In examples, the body 210 is formed from a coarse, braided material 230, which may be a cylindrical, helically wound braid, such as the common biaxial braid. In examples, the body 210 may have a three-dimensional tubular shape with a closed first end 214 and either a closed or open second end 218 forming a lumen 228 that is changeable in shape. Exemplary non-limiting dimensions of the body 210 are in the range of about 10 mm to about 30 mm in length, and about 1 mm to about 6 mm in width or diameter. However, sizes of the suture 212 and the length and diameter of the body 210 may vary.

In FIG. 2A, the soft anchor 200 is shown in a first, pre-deployed state. Pulling the suture 212 along a longitudinal axis L lengthens and narrows the body 210. The length of the body 210 is gained by reducing the angle between the threads of the braided material 230 at the crossing points of the threads so that the threads align mostly parallel, which also reduces the radial distance between opposing sides and hence the overall circumference. When the suture 212 is pulled transverse to the longitudinal axis L, the opposite action occurs, and the braided material 230 longitudinally contracts axially and expands radially in a secondary, deployed state, in this case by increasing the angle between the threads, as shown in FIG. 2B. In examples, the longitudinal axis L of the body 210 is generally aligned with the insertion direction of the soft anchor 200 and does not alter in orientation when in the secondary deployed state. The braided material 230 of the soft anchor 200 provides an advantage in that the body 210 can collapse and elongate naturally due to the alignment of the threads. Other non-limiting examples of soft anchors 200 are described in U.S. Patent No. 9,962,149 to ArthroCare Corporation (Austin, TX).

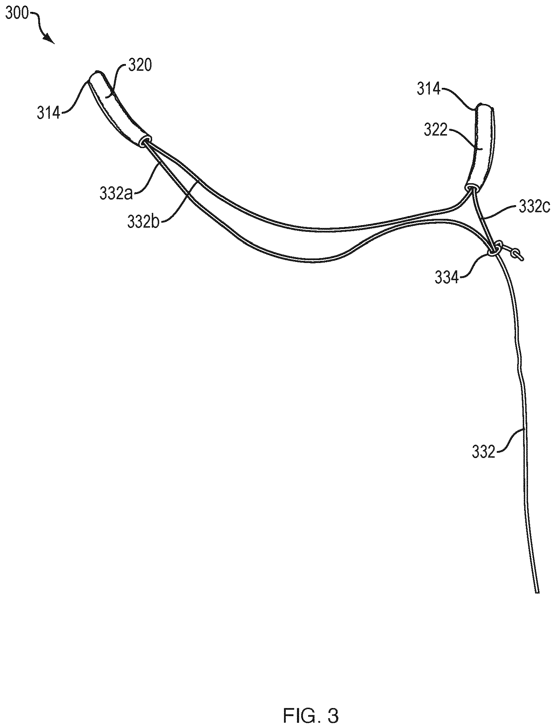

Turning now to FIG. 3, an example of an anchor/suture construct 300 according to the claimed invention for use in a tissue repair assembly, such as the tissue repair assembly 100 of FIG. 1, is shown. The anchor/suture construct 300 is designed to replace the anchor/suture construct of FIG. 1C in the tissue repair assembly 100. However, in examples (not shown), the distal end 111 of the needle 104 of the tissue repair assembly 100 may be modified to facilitate the expulsion of the construct 300 from the needle 104. For example, the slot 118 may be omitted from the needle 104, and a retaining element, such as a dimple, may be added to the bore 116 of the needle 104 to prevent the anchors of the anchor/suture construct 300 from being prematurely expelled from the needle 104.

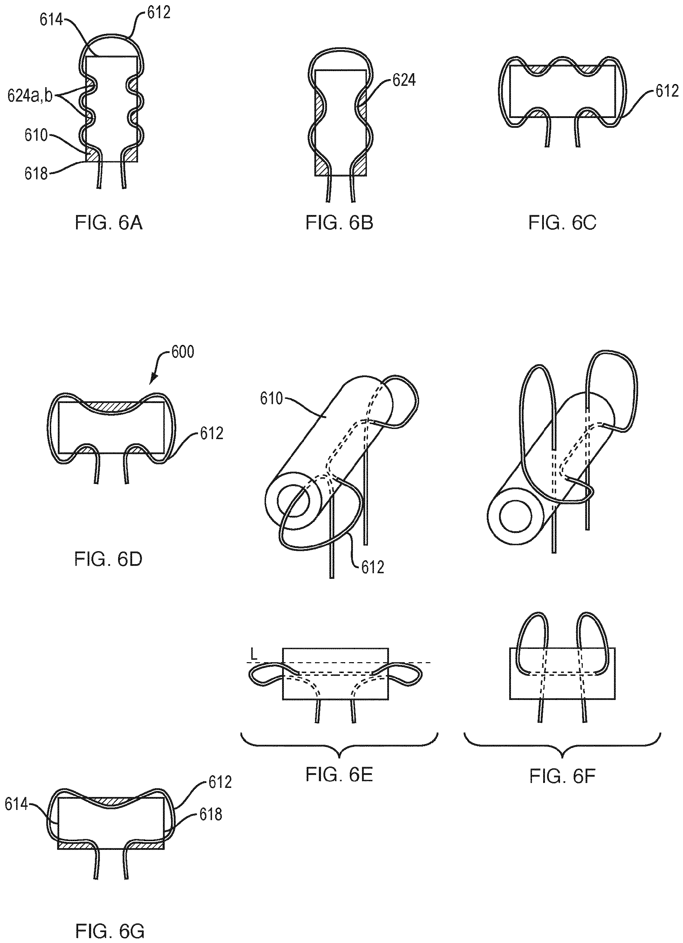

Still referring to FIG. 3, in examples, the anchor/suture construct 300 comprises a first soft anchor 320 and a second soft anchor 322. Each of the anchors 320, 322 may be constructed in the manner of the soft anchor 200 described above. In examples, a knotted suture 332 may be slidably woven longitudinally through opposite sides of the anchors 320, 322 and around the first end 314, as described further with regard to FIGS. 6A and 6B. Specifically, the suture 332 comprises a first section 332a slidably woven up a first side of the first soft anchor 320 and around the first end 314, and a second section 332b slidably woven down a second side of the first soft anchor 320 opposite the first side. The second section 332b exits the first soft anchor 320 and follows the same pattern through the second soft anchor 322. That is, the second section 332b is slidably woven up a first side of the second soft anchor 322 and around the first end 314, and a third section 332c is slidably woven down a second side of the second soft anchor 322 opposite the first side. The sliding knot 334 is formed between the first section 332a and the third section 332c. When the anchors 320, 322 are deployed in a patient's body and the sliding knot 334 is reduced toward the anchors 320, 322, the suture 332 slides through the anchors 320, 322, which function like pulleys. The anchors 320, 322 are compressed longitudinally, causing the anchors 320, 322 to expand radially and form a ball shape or cluster (FIG. 2B), thus increasing the contact area against the tissue. In examples, an additional sliding knot may be disposed at a location where the suture 332 exits the anchors 320, 322 to lock the anchors 320, 332 in the deployed shape, as further described below. It is also contemplated by this disclosure that one of the first soft anchor 320 or the second soft anchor 322 is formed from a hard material, such as PEEK or metal. However, use of two soft anchors 200 advantageously eliminates metal or PEEK in the body.

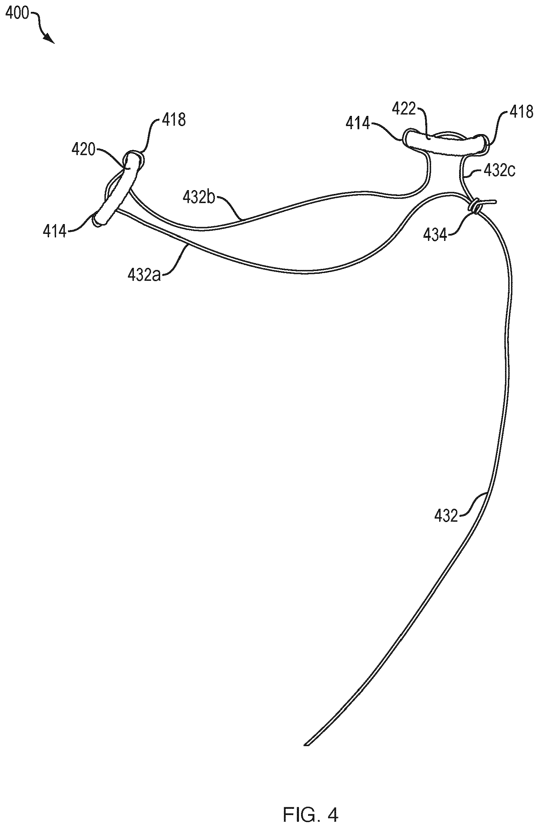

An alternative example of the anchor/suture construct 400 of this disclosure is shown in FIG. 4. In this example, the anchor/suture construct 400 also comprises a first soft anchor 420 and a second soft anchor 422. In examples, a knotted suture 432 may be slidably woven longitudinally through opposite sides of the anchors 420, 422 and around both the first end 414 and the second end 418, as described further below with regard to FIGS. 6C and 6D. Specifically, the suture 432 comprises a first section 432a slidably woven along a first side of the first soft anchor 420 and around the first end 414, and a second section 432b slidably woven along a second side of the first soft anchor 420 and around the second end 418 opposite the first end 414. The second section 432b exits the first side of first soft anchor 420 and follows the same pattern through the second soft anchor 422. That is, the second section 432b is slidably woven along the first side of the second soft anchor 422 and around the first end 414, and a third section 432c is slidably woven along a second side of the second soft anchor 422 and around a second end 418 opposite the first end 414. The sliding knot 434 is formed between the first section 432a and the third section 432c. When deployed and tensioned, the anchors 420, 422 expand radially to form a shape similar to that of a computer mouse (not shown).

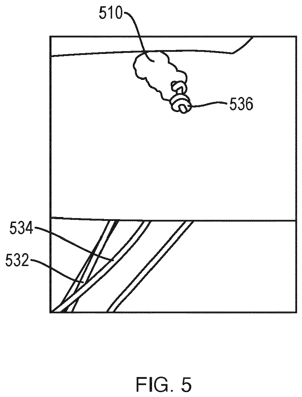

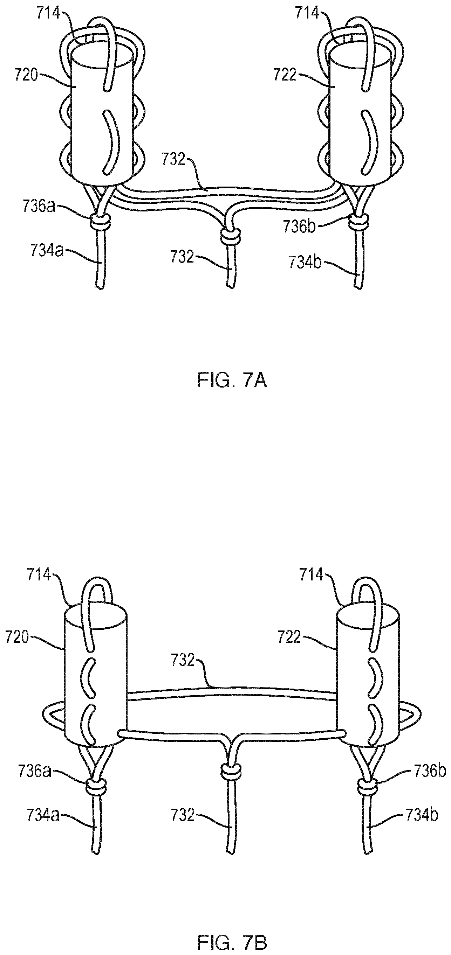

In another example, shown in FIG. 5, multiple sutures may be used to deploy and tension each of the anchor bodies 510 individually. For example, a separate suture 534 may be woven into an anchor body 510 to compress it independently of the connecting suture 532. One or both of the sutures 532, 534 may feature one or more additional sliding knots 536 to lock the anchor body 510 in tension. For example, the additional sliding knot 536 may be placed on the connecting suture 532 or on the separate tensioning suture 534, as shown. Further examples of suture/anchor constructs using a tensioning suture 534 are illustrated in FIGS. 7A and 7B.

Turning now to FIGS. 6A-G, various examples of forming a suture/anchor construct 600 of this disclosure by stitching a suture 612 through a textile body 610 are shown. In the example of FIG. 6A, the suture 612 may be stitched longitudinally through opposite sides of the body 610 such that two stitches 624a,b are formed on each side of the body 610 and the suture passes around the first end 614 of the body 610. In the example of FIG. 6B, the suture 612 may be stitched longitudinally through opposite sides of the body 610 such that a single stitch 624 is formed on each side of the body 610 and the suture passes around the first end 614 of the body 610. In the example of FIG. 6C, the suture 612 may be stitched longitudinally though the body 610 so that two stitches are formed on a first side and two stitches are formed on a second side of the body 610, and the suture 612 passes around both the first end 614 and the second end 618 of the body 610. In the example of FIG. 6D, the suture 612 may be stitched longitudinally though the body 610 so that two stitches are formed on a first side and a single stitch is formed on a second side of the body 610, and the suture passes around the first end 614 and the second end 618 of the body 610. In the examples of FIGS. 6E and 6F, the suture 612 may be stitched through the body 610 in two different planes (that is, both along the longitudinal axis L and transverse to the longitudinal axis L). In the example of FIG. 6G, the suture 612 may be stitched through the body 610 in a manner similar to that of FIG. 6D, except that the suture 612 passes through the open ends 614, 618 of the body 610. It will be appreciated that the suture 612 could be stitched through the body 610 in other ways not shown in FIGS. 6A-G. For example, the suture 612 could be stitched through the center of the body 610.

Turning now to FIGS. 7A and 7B, various examples of forming a suture/anchor construct 700 according to the claimed invention by stitching a connecting suture 712 and a tensioning suture 734a,b through a distal anchor 720 and a proximal anchor 722 are shown. In the example of FIGS. 7A and 7B, the connecting suture 732 may be stitched through the distal and proximal anchors 720, 722 in a manner described above with regard to FIGS. 6A-G. A first tensioning suture 734a is also stitched longitudinally through opposing sides of the distal anchor 720 such that it passes around the first end 714. A second tensioning suture 734b is stitched longitudinally through opposing sides of the proximal anchor 722 such that it passes around the first end 714. The tensioning sutures 734a,b are used for separately deploying the distal and proximal anchors 720, 722 independent of the connecting suture 732. Additional sliding knots 736a,b may be placed on the tensioning sutures 734a,b for locking the distal and proximal anchors 720, 722 in the deployed position.

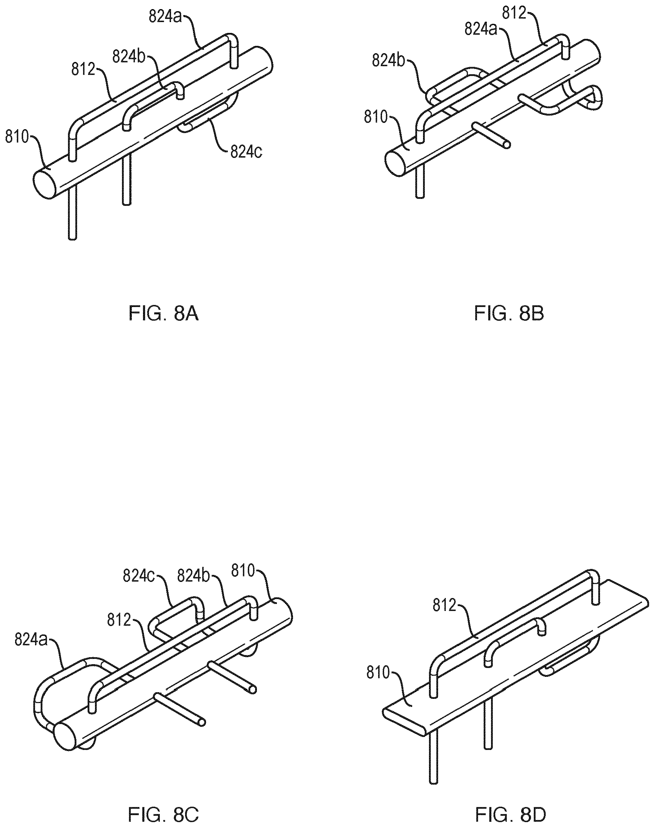

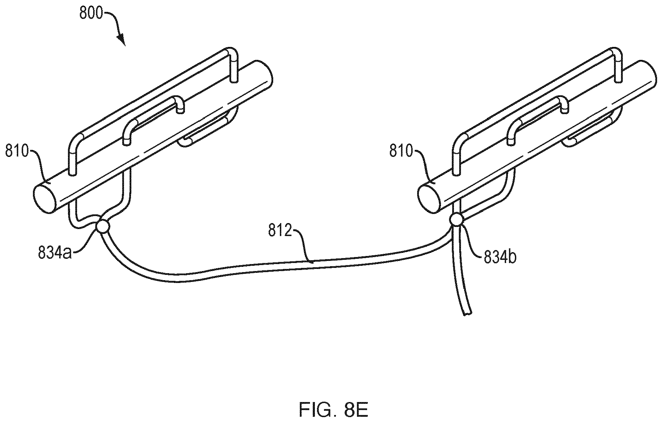

Turning now to FIGS. 8A-D, additional examples of forming a suture/anchor construct 800 of this disclosure by stitching a suture 812 through a textile body 810 are shown. In the example of FIG. 8A, the suture 812 may be stitched longitudinally through opposite sides of the body 810 such that two overlapping stitches 824a,b are formed on a first side of the body 810 and a third stitch 824c is formed on the second side of the body 810 opposite the first side. Both ends of the suture 812 exit the second side of the body 810. In the example of FIG. 8B, the suture 812 may be stitched longitudinally through a first side of the body 810 to form first stitch 824a and through a second side of the body 810 to form second stitch 824b at an angle that is substantially 90° from the first stitch 824a. Thus, the two ends of the suture 812 exit the body 810 at a 90° angle from one another. In the example of FIG. 8C, the suture 812 may be stitched longitudinally through a first side of the body 810 to form first stitch 824a and through a second side of the body to form second stitch 824b at an angle that is substantially 90° from the first stitch 824a. A third stitch 824c is then formed through the first side of the body 810 spaced apart from the first stich 824a. The two ends of the suture 812 exit the body 810 at a 90° angle from the second stitch 824b. FIG. 8D shows an example similar to that of FIG. 8A, except that the body 810 is flat, rather than tubular. The suture/anchor construct 800 using, for example, the stitching pattern on body 810 of FIG. 8A, is shown in FIG. 8E. In the suture/anchor construct 800, two sliding knots 834a,b are formed in the suture 812 adjacent each body 810 for separately deploying the textile bodies 810. When deployed, the textile bodies of FIGS. 8A-E will fold, rather than expand radially. In other examples of the suture/anchor construct 800, not shown, the stitching pattern on the textile bodies 810 need not be the same.

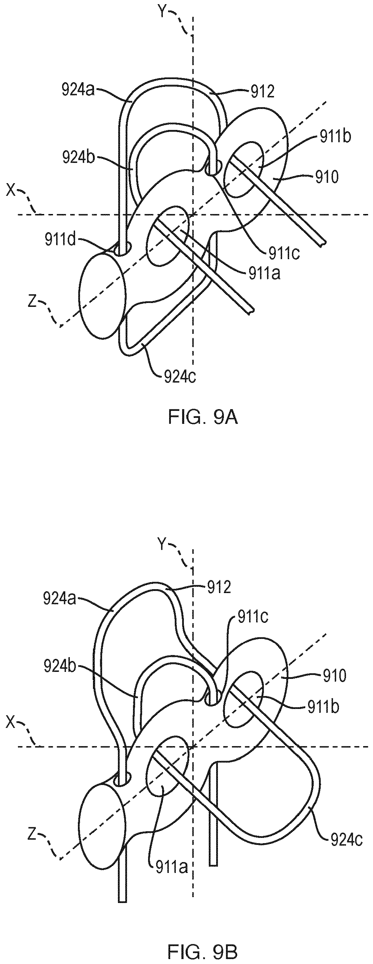

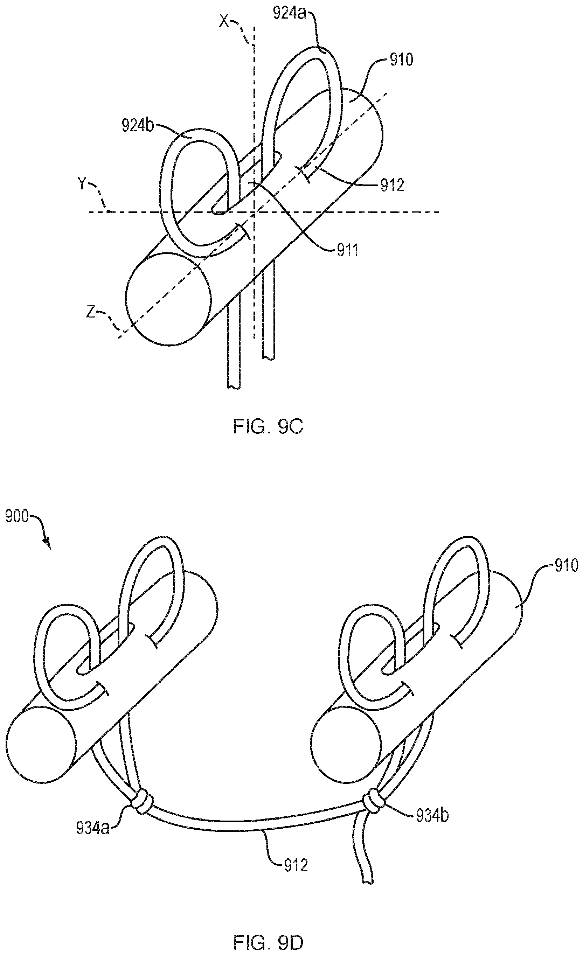

Turning now to FIGS. 9A-C, additional examples of forming a suture/anchor construct 900 of this disclosure by stitching a suture 912 through a textile body 910 are shown. In the example of FIG. 9A, four bifurcations in the body 910 form eyelets 911a,b,c,d in two perpendicular axes X, Y. It will be appreciated that sizes of the bifurcations and the eyelets 911a,b,c,d can vary. The suture 912 may pass through the eyelets 91 1a,b,c,d and through the body 910 such that two overlapping stitches 924a,b are formed on a first side of the body 910 and a third stitch 924c is formed on the second side of the body 910 opposite the first side. Both ends of the suture 912 pass through the eyelets 911a,b along a third axis Z. In the example of FIG. 9B, the suture 912 may pass through the eyelets 911a,b,c,d and through the body 910 such that two overlapping stitches 924a,b are formed on a first side of the body 910 and a third stitch 924c is formed on the second side of the body 910 at an angle that is substantially 90° from the first and second stitch 924a,b. Both ends of the suture 912 pass through the eyelets 911c,d along the first axis X. In the example of FIG. 9C, the bifurcations in the body 910 form a single eyelet 911. The suture 912 may pass through the eyelet 911 and through the body 910 such that two non-overlapping stitches 924a,b are formed on a first side of the body and both ends of the suture 912 pass through the eyelet 911 along the second axis Y. The suture/anchor construct 900 using, for example, the stitching pattern on body 910 of FIG. 9C, is shown in FIG. 9D. In the suture/anchor construct 900, two sliding knots 934a,b are formed in the suture 12 adjacent each body 910 for separately deploying the textile bodies 910. In other examples of the suture/anchor construct 900, not shown, the stitching pattern on the textile bodies 910 need not be the same.

Scope of the disclosure is thus indicated by the appended claims, rather than by the foregoing description.

## Claims

1. A tissue repair assembly comprising:
a needle; and
a suture/anchor construct (300) disposed within a bore of the needle, the suture/anchor construct (300) comprising:
first and second flexible anchors (320, 322) coupled to a suture (332), wherein each anchor is a hollow, cylindrical woven braid, the suture (332) comprising:
a first section (332a) slidably woven up a first side of the first anchor (320) and around a first end (314) of the first anchor (320);
a second section (332b) slidably woven down a second side of the first anchor (320) opposite the first side, exiting the first anchor (320) and woven up a first side of the second anchor (322) and around a first end (3140) of the second anchor (322);
a third section (332c) slidably woven down a second side of the second anchor (322), opposite the first side; and
at least one knot (334) formed between the first section (332a) and the third section (332c);
wherein the first and second anchors (320, 322) are deployable from a first configuration to a second configuration within a surgical site when tension is applied to the first knot (324).

2. The assembly of claim 1, further comprising an additional knot formed in the suture for maintaining at least one of the first and second flexible anchors (320, 322) in the second configuration.

3. The assembly of claim 1, wherein, in the first configuration, the first and second anchors (320, 322) are elongate and, in the second configuration, the first and second anchors (320, 322) are compressed axially and extended radially.

4. A suture/anchor construct comprising:
first and second flexible anchors (720, 722) coupled to a first suture (732),
wherein each anchor (720, 722) is a hollow, cylindrical woven braid, the first suture (732) comprising:
a first section slidably woven through the first anchor (720);
a second section slidably woven at least partially through the first anchor (720) and at least partially through the second anchor (722); and
a third section slidably woven through the second anchor (722);
a second suture (734a) slidably woven up a first side of the first anchor (720), around a first end (714) of the first anchor (720) and down a second side of the first anchor (729) opposite the first side; and
a third suture (734b) slidably woven up a first side of the second anchor (722), around a first end (714) of the second anchor (722) and down a second side of the second anchor (722) opposite the first side;
wherein the first and second anchors (720, 724) are deployable from a first configuration to a second configuration within a surgical site when tension is applied to the second and third sutures (734a, 734b), respectively.

5. The construct of claim 4, further comprising a first knot formed between the first section and the third section of the first suture.

6. The construct of claim 4, wherein the second suture (734a) comprises a second knot (736a) for maintaining the first anchor (720) in the second configuration.

7. The construct of claim 4, wherein the third suture (734b) comprises a third knot (736b) for maintaining the second anchor (722) in the second configuration.

8. The construct of claim 4, wherein, in the first configuration, the first and second anchors (720, 722) are elongate and, in the second configuration, the first and second anchors (720, 722) are compressed axially and extended radially.

## Patentansprüche

1. Gewebereparaturanordnung, umfassend:
eine Nadel und
ein Nahtfaden/Anker-Konstrukt (300), das in einer Bohrung der Nadel angeordnet ist, wobei das Nahtfaden/Anker-Konstrukt (300) Folgendes umfasst:
einen ersten und einen zweiten flexiblen Anker (320, 322), die an einen Nahtfaden (332) gekoppelt sind, wobei jeder Anker ein hohles, zylindrisches Webgeflecht ist, wobei der Nahtfaden (332) Folgendes umfasst:
einen ersten Abschnitt (332a), der verschiebbar an einer ersten Seite des ersten Ankers (320) hinauf und um ein erstes Ende (314) des ersten Ankers (320) herum gewebt ist,
einen zweiten Abschnitt (332b), der verschiebbar an einer der ersten Seite gegenüberliegenden zweiten Seite des ersten Ankers (320) hinunter gewebt ist und aus dem ersten Anker (320) austritt und an einer ersten Seite des zweiten Ankers (322) hinauf um ein erstes Ende (3140) des zweiten Ankers (322) herum gewebt ist,
einen dritten Abschnitt (332c), der verschiebbar an einer der ersten Seite gegenüberliegenden zweiten Seite des zweiten Ankers (322) hinunter gewebt ist, und
mindestens einen Knoten (334), der zwischen dem ersten Abschnitt (332a) und dem dritten Abschnitt (332c) gebildet ist,
wobei der erste und der zweite Anker (320, 322) in einem operativen Situs aus einer ersten Konfiguration in eine zweite Konfiguration ausbringbar sind, wenn der erste Knoten (324) mit Spannung beaufschlagt wird.

2. Anordnung nach Anspruch 1, ferner umfassend einen zusätzlichen in dem Nahtfaden gebildeten Knoten, um den ersten und/oder den zweiten Ankers (320, 322) in der zweiten Konfiguration zu halten.

3. Anordnung nach Anspruch 1, wobei der erste und der zweite Anker (320, 322) in der ersten Konfiguration länglich sind und der erste und der zweite Anker (320, 322) in der zweiten Konfiguration axial komprimiert und radial erweitert sind.

4. Nahtfaden/Anker-Konstrukt, umfassend:
einen ersten und einen zweiten flexiblen Anker (720, 722), die an einen ersten Nahtfaden (732) gekoppelt sind, wobei jeder Anker (720, 722) ein hohles, zylindrisches Webgeflecht ist, wobei der erste Nahtfaden (732) Folgendes umfasst:
einen ersten Abschnitt, der verschiebbar durch den ersten Anker (720) gewebt ist,
einen zweiten Abschnitt, der mindestens teilweise durch den ersten Anker (720) und mindestens teilweise durch den zweiten Anker (722) gewebt ist, und
einen dritten Abschnitt, der verschiebbar durch den zweiten Anker (722) gewebt ist,
einen zweiten Nahtfaden (734a), der verschiebbar an einer ersten Seite des ersten Ankers (720) hinauf, um ein erstes Ende (714) des ersten Ankers (720) herum und an einer der ersten Seiten gegenüberliegenden zweiten Seite des ersten Ankers (729) hinunter gewebt ist, und
einen dritten Nahtfaden (734b), der verschiebbar an einer ersten Seite des zweiten Ankers (722) hinauf, um ein erstes Ende (714) des zweiten Ankers (722) herum und an einer der ersten Seiten gegenüberliegenden zweiten Seite des zweiten Ankers (722) hinunter gewebt ist,
wobei der erste und der Anker (720, 724) in einem operativen Situs aus einer ersten Konfiguration in eine zweite Konfiguration ausbringbar sind, wenn der zweite bzw. der dritte Nahtfaden (734a, 734b) mit Spannung beaufschlagt werden.

5. Konstrukt nach Anspruch 4, ferner umfassend einen ersten Knoten, der zwischen dem ersten Abschnitt und dem dritten Abschnitt des ersten Nahtfadens gebildet ist.

6. Konstrukt nach Anspruch 4, wobei der zweite Nahtfaden (734a) einen zweiten Knoten (736a) zum Halten des ersten Ankers (720) in der zweiten Konfiguration umfasst.

7. Konstrukt nach Anspruch 4, wobei der dritte Nahtfaden (734b) einen dritten Knoten (736b) zum Halten des zweiten Ankers (722) in der zweiten Konfiguration umfasst.

8. Konstrukt nach Anspruch 4, wobei der erste und der zweite Anker (720, 722) in der ersten Konfiguration länglich sind und der erste und der zweite Anker (720, 722) in der zweiten Konfiguration axial komprimiert und radial erweitert sind.

## Revendications

1. Ensemble de réparation de tissu comprenant :
une aiguille ; et
une construction de suture/ancrage (300) disposée dans un alésage de l'aiguille, la construction de suture/ancrage (300) comprenant :
des première et deuxième ancres flexibles (320, 322) couplée à une suture (332), chaque ancre étant une tresse tissée creuse et cylindrique, la suture (332) comprenant :
une première section (332a) tissée de manière coulissante vers le haut le long d'un premier côté de la première ancre (320) et autour d'une première extrémité (314) de la première ancre (320) ;
une deuxième section (332b) tissée de manière coulissante vers le bas le long d'un deuxième côté de la première ancre (320) opposé au premier côté, sortant de la première ancre (320) et tissée vers le haut le long d'un premier côté de la deuxième ancre (322) et autour d'une première extrémité (3140) de la deuxième ancre (322) ;
une troisième section (332c) tissée de manière coulissante vers le bas le long d'un deuxième côté de la deuxième ancre (322), à l'opposé du premier côté ; et
au moins un noeud (334) formé entre la première section (332a) et la troisième section (332c) ;
les première et deuxième ancres (320, 322) pouvant être déployées d'une première configuration à une deuxième configuration à l'intérieur d'un site chirurgical lorsqu'une tension est appliquée au premier noeud (324).

2. Ensemble selon la revendication 1, comprenant en outre un noeud supplémentaire formé dans la suture pour maintenir au moins l'une des premières et deuxièmes ancres flexibles (320, 322) dans la deuxième configuration.

3. Ensemble selon la revendication 1, dans la première configuration, les première et deuxième ancres (320, 322) étant allongées et, dans la deuxième configuration, les première et deuxième ancres (320, 322) étant comprimées axialement et étendues radialement.

4. Construction de suture/ancrage comprenant :
des première et deuxième ancres flexibles (720, 722) couplée à une première suture (732), chaque ancre (720, 722) étant une tresse tissée creuse et cylindrique, la première suture (732) comprenant :
une première section tissée de manière coulissante à travers la première ancre (720) ;
une deuxième section tissée de manière coulissante au moins partiellement à travers la première ancre (720) et au moins partiellement à travers la deuxième ancre (722) ; et
une troisième section tissée de manière coulissante à travers la deuxième ancre (722) ;
une deuxième suture (734a) tissée de manière coulissante vers le haut le long d'un premier côté de la première ancre (720), autour d'une première extrémité (714) de la première ancre (720) et vers le bas le long d'un deuxième côté de la première ancre (729) opposé au premier côté ; et
une troisième suture (734b) tissée de manière coulissante vers le haut le long d'un premier côté de la deuxième ancre (722), autour d'une première extrémité (714) de la deuxième ancre (722) et vers le bas le long d'un deuxième côté de la deuxième ancre (722) opposé au premier côté ;
les première et deuxième ancres (720, 724) pouvant être déployées d'une première configuration à une deuxième configuration à l'intérieur d'un site chirurgical lorsqu'une tension est appliquée aux deuxième et troisième sutures (734a, 734b), respectivement.

5. Construction selon la revendication 4, comprenant en outre un premier noeud formé entre la première section et la troisième section de la première suture.

6. Construction selon la revendication 4, la deuxième suture (734a) comprenant un deuxième noeud (736a) pour maintenir la première ancre (720) dans la deuxième configuration.

7. Construction selon la revendication 4, la troisième suture (734b) comprenant un troisième noeud (736b) pour maintenir la deuxième ancre (722) dans la deuxième configuration.

8. Construction selon la revendication 4, dans la première configuration, les première et deuxième ancres (720, 722) étant allongées et, dans la deuxième configuration, les première et deuxième ancres (720, 722) étant comprimées axialement et étendues radialement.
